# EUROPÄISCHE PATENTANMELDUNG

(11) **EP 3 064 159 A1**
(43) Veröffentlichungstag der Anmeldung: **07.09.2016**
(21) Anmeldenummer: 16163296.3
(22) Anmeldetag: 10.02.2005
(51) Int. Cl.: A61B 17/56, A61B 5/00, A61F 2/30

(54) **BAUTEIL UND VERFAHREN ZUM ZUSAMMENBAU EINER IMPLANTATANORDNUNG**

(30) Priorität: 10.02.2004 DE 102004006501
(62) Teilanmeldung aus: 05714957.7
(71) Anmelder: Synthes GmbH, 4436 Oberdorf (CH)
(72) Erfinder: DUDA, Georg, 12209 Berlin (DE); HELLER, Markus, 10115 Berlin (DE)
(74) Vertreter: Klunker . Schmitt-Nilson . Hirsch

(57) **Zusammenfassung**

Die Erfindung betrifft ein Bauteil zum Anordnen an einem Implantat sowie ein Verfahren zum Zusammenbau einer Implantatanordnung. Das Bauteil umfaßt ein Grundbauteil, mindestens eine in dem Grundbauteil angeordnete Sensoreinrichtung zum Erfassen einer Meßgröße und zum Erzeugen von Meßdaten für die erfaßte Meßgröße, eine in dem Grundbauteil angeordnete Telemetrieeinrichtung zum Senden und Empfangen von Daten und eine Datenübertragungsverbindung zwischen der mindestens einen Sensoreinrichtung und der Telemetrieeinrichtung zum Übertragen von Daten zwischen der mindestens einen Sensoreinrichtung und der Telemetrieeinrichtung, wobei die Daten die Meßdaten umfassen. An dem Grundbauteil sind Montagemittel zum Montieren des Grundbauteils in einer Implantatausnehmung gebildet.

## Beschreibung

Die Erfindung liegt auf dem Gebiet der Technologie von Implantaten.

Implantate werden sowohl beim Menschen als auch bei Tieren genutzt, um Funktionen des Knochenskeletts zu unterstützen oder sogar Teile des Skeletts zu ersetzen. Zu den Implantaten, die Funktionen des Skeletts unterstützen und deshalb auch als Stützimplantate bezeichnet werden können, gehören Platten, Schienen oder winkelstabile Systeme aus einem festen Material, beispielsweise Edelstahl oder Titan, die an Skelettknochen befestigt werden, um deren Stützfunktion zu übernehmen. Derartige Stützimplantate werden beispielsweise im Zusammenhang mit Knochenbrüchen verwendet, um Knochenteile während des Heilungsprozesses relativ zueinander zu fixieren. Hierbei werden die Implantate beispielsweise mit Hilfe von Schrauben an den Knochen befestigt. Um bei Knochenfrakturen oder in der plastischen Chirurgie die Knochenflächen zusammenzufügen, werden spezielle Implantate verwendet, zum Beispiel Platten oder winkelstabile Systeme. Zur Fixierung der Knochen werden die Knochenfragmente insbesondere mittels Schrauben oder Bolzen mit den Platten verbunden. Um eine Anpassung der Osteosynthese an die jeweilige Patientensituation zu erlauben, sind die Platten mit mehreren Öffnungen zur Aufnahme der Schrauben oder Bolzen versehen.

Die Heilung, beispielsweise die Heilung eines Knochenbruchs, ist ein biologischer Prozeß, dessen Verlauf von zahlreichen biologischen aber auch bio-mechanischen Umgebungsfaktoren abhängt. Es gibt Vorschläge, die Heilung durch systemische oder lokale Applikation stimulierender Substanzen zu unterstützen. Die Freisetzung entsprechender Substanzen im zeitlichen Verlauf und die Auswirkungen auf relevante bio-physikalische und/oder bio-chemische Parameter am Ort des Interesses kann heute nur unzureichend beurteilt werden. Unabhängig von erfolgter/nicht-erfolgter Unterstützung wird derzeit der Fortschritt des Heilungsprozesses anhand von Verfahren beurteilt, die mit schwerwiegenden Nachteilen behaftet sind.

Die Auswertung von Röntgenbildern ist das Standardverfahren zur Dokumentation des Heilungsfortschrittes bei der Knochenbruchheilung. Dieses Verfahren ist jedoch mit einer Strahlenbelastung für den Patienten verbunden und somit ein invasives Verfahren, bei dem darüber hinaus eine objektivierbare Dokumentation des Heilungsforschrittes nur beschränkt möglich ist. Die Entscheidung, wann der Heilungsprozeß soweit abgeschlossen ist, daß beispielsweise ein temporär eingebrachtes Implantat wieder entfernt werden kann, wird daher wesentlich von der Erfahrung des Chirurgen bestimmt. Verfahren zur Beurteilung des Heilungsfortschrittes auf Basis von Computer-Tomographie sind aufgrund der Strahlungsbelastung ebenfalls invasive Verfahren. Bei liegendem Implantat ist die Bewertung in der Regel schwierig, aber auch zeit- und ressourcenintensiv und mit sehr hohen Kosten verbunden, so daß eine routinemäßige Anwendung nicht möglich ist. Ähnliches gilt für MR basierte Verfahren, die zeitintensiv, bei liegendem Implantat nicht immer anwendbar oder von begrenzter Aussagekraft, kostenintensiv und nicht in großer Zahl einsetzbar sind. Bei speziellen Verfahren zur Stabilisierung von Frakturen, zum Beispiel mittels sogenannter "Fixateur externe", sind nicht-invasive Verfahren anwendbar, welche die Veränderung in der Relativbewegung der Knochenfragmente mit Hilfe optischer Messungen berührungslos dokumentieren (G. N. Duda, B. Bartmeyer, S. Sporrer, W. R. Taylor, M. Raschke, N. D. Haas; "Does partial weight bearing unload a healing bone in external ring fixation?"; Langenbecks Arch Surg., Oktober 2003, 388(5), 298-304.) und somit den Verlauf der Heilung darstellen können. Für Stabilisierungsverfahren, bei welchen die Implantate, beispielsweise Platten, winkelstabile "Fixateur interne", unter der Haut liegen und die Befestigungselemente einer optischen Messung nicht zugänglich sind, ist das zuvor beschriebene berührungslose Verfahren nicht anwendbar.

US 6,034,296 beschreibt ein Sensor-System zur Befestigung an einem Implantat. Das Sensor-System ist mit einem Druck-Meßsystem ausgestattet, um die im Implantat auftretenden mechanischen Spannungen im alltäglichen Gebrauch zu messen. Dazu ist das Sensor-System mit Hilfe von Schrauben oder mittels geschweißter Verbindungen an dem Implantat befestigt. Außerdem umfaßt das Sensor-System eine Telemetreieinheit, um die gemessenen Werte an einen externen Empfänger zu senden.

DE 198 58 889 A1 offenbart ein Implantat zur Fixierung von Knochen. Als eine Ausführungsform wird vorgeschlagen, an dem Implantat einen Sensor für die Ermittlung der auf das Implantat einwirkenden Kräfte sowie eine Telemetrieeinheit für die Übermittlung der Meßergebnisse an eine externe Einheit anzuordnen.

Aufgabe der Erfindung ist es, ein verbessertes Bauteil anzugeben, daß in Verbindung mit einem Implantat nutzbar ist, so daß die Erfassung medizinisch relevanter Meßgrößen erleichtert wird. Darüber hinaus soll die Montage des Bauteils möglichst einfach sein.

Diese Aufgabe wird erfindungsgemäß durch ein Bauteil nach Anspruch 1, ein Stützimplantat nach Anspruch 13 sowie ein Ersatzimplantat nach Anspruch 17.

Die Erfindung umfaßt den Gedanken, ein Bauteil zum Anordnen an einem Implantat zur Verfügung zu stellen, wobei das Bauteil die folgenden Merkmale aufweist: ein Grundbauteil, mindestens eine in dem Grundbauteil angeordnete Sensoreinrichtung zum Erfassen einer Meßgröße und zum Erzeugen von Meßdaten für die erfaßte Meßgröße, eine in dem Grundbauteil angeordnete Telemetrieeinrichtung zum Senden und/oder Empfangen von Daten und eine Datenübertragungsverbindung zwischen der mindestens einen Sensoreinrichtung und der Telemetrieeinrichtung zum Übertragen von Daten zwischen der mindestens einen Sensoreinrichtung und der Telemetrieeinrichtung, wobei die Daten die Meßdaten umfassen und wobei an dem Grundbauteil Montagemittel lösbaren Montieren des Grundbauteils in einer Implantatausnehmung eines Implantats gebildet sind.

Auf diese Weise wird ein Bauteil zur Verfügung gestellt, das auf einfache Weise an dem zu implantierenden Implantat montiert werden kann. Die Montage des Grundbauteils mit der mindestens einen Sensoreinrichtung und der Telemetrieeinrichtung an dem Implantat hat den Vorteil, daß hierdurch in räumlicher Nähe zu dem Implantat, welches seinerseits im Körper des Lebewesens an einem Ort plaziert ist, an dem ein zu beobachtender Prozeß, beispielsweise ein Heilungsprozeß, abläuft, mit Hilfe der Sensoreinrichtung Meßdaten erfaßt werden können, die dann nach einer Übertragung von der Sensoreinrichtung an die Telemetrieeinrichtung von außerhalb des Körpers des Lebewesens von der Telemetrieeinrichtung mittels Auslesen von Daten abgerufen ("passive Ausführung", zum Beispiel mittels einer Transponder-Technik) oder von der Telemetrieeinrichtung gesendet ("aktive Ausführung") und in einer Auswerteeinrichtung ausgewertet werden können. Die mit Hilfe der vorgeschlagenen konstruktiven Ausgestaltung des Bauteils ermöglichte Integration von Sensoreinrichtung und Telemetrieeinrichtung an dem Implantat unterstützt eine räumlich möglichst nahe Meßwerterfassung zu dem Ort, an dem Änderungen im Verlauf des Heilungsprozesses beobachtet werden können. So ermöglicht diese Konstruktion des Bauteils nach Einbau des Bauteils in das Implantat beispielsweise die Überwachung von Frakturheilungen. Sensoreinrichtungen, die als solche dem Fachmann bekannt sind, können auf diese Art und Weise mit geringem Aufwand an einem Implantat angeordnet werden. Die mit Hilfe der Sensoreinrichtung ausgeführte Messung betrifft beispielsweise eine pH-Wert-Messung, eine Sauerstoffpartialdruck-Messung, eine Druckmessung (Flüssigkeitsdruck), eine Beschleunigungsmessung und/oder eine Proteinkonzentrationsmessung.

Eine zweckmäßige Ausgestaltung der Erfindung kann vorsehen, daß das Bauteil in einer Implantatausnehmung angeordnet ist. Hierdurch wird die feste Lokalisierung des Bauteils an dem Implantat erleichtert.

Eine mit geringem Aufwand ausführbare Möglichkeit zum Montieren des Grundbauteils am Implantat ist bei einer bevorzugten Ausgestaltung der Erfindung dadurch geschaffen, daß die Montagemittel einen Montageabschnitt zum wenigstens teilweisen Einführen in die Implantatausnehmung umfassen. Das wenigstens teilweise Einführen des Montageabschnitts des Grundbauteils sichert eine stabile Halterung des Grundbauteils an dem Implantat.

Bei einer Ausgestaltung der Erfindung kann vorgesehen sein, daß im Bereich des Montageabschnitts ein Gewindeabschnitt zum Einschrauben des Grundbauteils in die Implantatausnehmung gebildet ist. Die mit Hilfe des Gewindeabschnitts ermöglichte Schraubbefestigung des Bauteils ermöglicht ein zuverlässiges Befestigen und Lösen des Bauteils an dem Implantat.

Ein Hindurchrutschen des Bauteils durch die Implantatausnehmung ist bei einer vorteilhaften Ausführungsform der Erfindung dadurch verhindert, daß das Grundbauteil im Längsschnitt einen im wesentlichen T-förmigen Querschnitt mit einem Kopfteil und einem Basisteil aufweist. Das im Vergleich zum Basisteil breitere Kopfteil verhindert, daß das Bauteil zu weit in die Implantatausnehmung hinein gelangt.

Bei einer Weiterbildung der Erfindung kann vorgesehen sein, daß die mindestens eine Sensoreinrichtung im Bereich eines Endabschnitts des Grundbauteils und die Telemetrieeinrichtung im Bereich eines gegenüberliegenden Endabschnitts des Grundbauteils angeordnet sind. Hierdurch wird erreicht, daß einerseits die mindestens eine Sensoreinrichtung möglichst nahe einem zu untersuchenden Ort angeordnet werden kann, an dem die Meßgröße erfaßt werden soll. Andererseits kann die Telemetrieeinrichtung bei dieser Ausgestaltung möglichst so angeordnet werden, daß ein störungsfreies Empfangen/Senden der Daten gewährleistet ist. Dieses wird bei einer weiteren Ausführungsform der Erfindung insbesondere dadurch unterstützt, daß die Telemetrieeinrichtung im wesentlichen in dem Kopfteil des Grundbauteils angeordnet ist. Das Kopfteil wird beim Anordnen des Bauteils an dem Implantat nicht in die Implantatausnehmung eingeführt, so daß es möglichst weitgehend freigelegt ist, was das ungestörte Senden/Empfangen von Daten erleichtert.

Bei einer zweckmäßigen Ausgestaltung der Erfindung ist vorgesehen, daß an dem Grundbauteil ein Aufnahmeraum zum Aufnehmen eines Wirkstoffs gebildet ist, wobei der Aufnahmeraum mit einer Öffnung zum Abgeben des Wirkstoffs nach außen in Verbindung steht. Hierdurch ist eine Möglichkeit geschaffen, einen medizinischen Wirkstoff zusammen mit dem Implantat zu implantieren, wobei der Wirkstoff in dem Aufnahmeraum untergebracht ist.

Zum kontrollierten Abgeben des Wirkstoffs aus dem Aufnahmeraum durch die Öffnung im Rahmen einer Wirkstoffapplikation ist bei einer vorteilhaften Ausgestaltung der Erfindung eine Abgabeeinrichtung vorgesehen. Die Abgabeeinrichtung kann eine Pumpeinrichtung zum Pumpen einer Menge des Wirkstoffs aus dem Aufnahmeraum durch die Öffnung umfassen. Darüber hinaus sieht eine bevorzugte Weiterbildung der Erfindung vor, daß die Abgabeeinrichtung einen Öffnungsmechanismus zum Öffnen/Schließen der Öffnung umfaßt. Auf diese Weise sind verschiedenen Ausführungsformen eines Bauteils gebildet, das dazu verwendet werden kann, sowohl Meßdaten zu erfassen und diese mit Hilfe der Telemetrieeinrichtung zu übertragen als auch einen Wirkstoff an einem Ort in Implantat Nähe zur Verfügung zu stellen. Mikromechanismen zum Bilden der Abgabeeinrichtung, einer Pumpe und/oder des Öffnungsmechanismus sind dem Fachmann als solche beispielsweise aus dem Bereich der Mikrosystemtechnik in medizinischen oder nicht-medizinischen Anwendungen bekannt.

Zweckmäßig ist bei einer Ausführungsform der Erfindung vorgesehen, daß die Abgabeeinrichtung über eine weitere Datenübertragungsverbindung zum Übertragen von Daten mit der Telemetrieeinrichtung verbunden ist. Hierdurch ist die Möglichkeit geschaffen, von außen elektronische Daten an die Abgabeeinrichtung zu übersenden und/oder elektronische Daten von der Abgabeeinrichtung über die Telemetrieeinrichtung zu empfangen. Hierdurch kann die Abgabe des Wirkstoffes von außerhalb des Körpers, in welchem das Implantat mit dem Bauteil verwendet wird, kontrolliert werden.

Darüber hinaus kann eine Fortbildung der Erfindung eine Steuereinheit vorsehen, die mit der mindestens einen Sensoreinrichtung und der Abgabeeinrichtung verbunden ist, um die Erfassung der Meßdaten mit Hilfe der mindestens einen Sensoreinrichtung und die Abgabe des Wirkstoffs mit Hilfe der Abgabeeinrichtung gemeinsam zu steuern. Eine gemeinsame Steuerung bedeutet in diesem Zusammenhang im wesentlichen eine aufeinander abgestimmte Steuerung, so daß das Erfassen von Meßdaten und das Abgeben des Wirkstoffs beispielsweise in zeitlicher Hinsicht miteinander korreliert werden können.

Das beschriebene Bauteil mit der mindestens einen Sensoreinrichtung, der Telemetrieeinrichtung und den Montagemitteln am Grundbauteil kann sowohl in einer Implantatausnehmung eines Stützimplantats, insbesondere einer Platte, eines "Nagels" (Marknagel) oder einer Schiene aus einem Material mit hoher Steifigkeit, als auch in einer Implantatausnehmung eines Ersatzimplantats, insbesondere eines künstlichen Hüft-, Knie- oder Schultergelenkts, angeordnet werden. Hierbei ist der Montageaufwand minimiert, wenn die Implantatausnehmung eine beim Implantieren nutzbare Montageausnehmung zum Aufnehmen von Implantat-Befestigungsmitteln ist. Derartige Montageausnehmungen sind in der Regel an Implantaten vorgesehen, um das Implantat mit Hilfe von Schrauben oder ähnlichen Befestigungsmitteln zu befestigen. Die Anordnung des Bauteils in einer solchen Montageaufnahme, die für die Befestigung des Implantats redundant ist, vermeidet, daß das Implantat zusätzlich mechanisch bearbeitet werden muß, um eine Anordnung des Bauteils zu ermöglichen. Eine einfache aber hinsichtlich der mechanischen Befestigung sichere Anordnung des Bauteils ist bei einer Ausgestaltung der Erfindung dadurch gewährleistet, daß die Implantatausnehmung einen Innengewindeabschnitt aufweist, in welche das Bauteil eingeschraubt ist.

Die Erfindung wird im folgenden anhand von Ausführungsbeispielen unter Bezugnahme auf eine Zeichnung näher erläutert. Hierbei zeigen:
- Figur 1: eine Anordnung mit einem Knochen und einem hieran befestigten Implantat;
- Figur 2: eine Querschnittsdarstellung der Anordnung nach Figur 1 entlang einer Linie AA';
- Figur 3: ein Bauteil zum Montieren an einem Implantat mit einer Sensoreinrichtung und einer Telemetrieeinrichtung im Querschnitt;
- Figur 4: ein weiteres Bauteil zum Montieren an einem Implantat mit einer Sensoreinrichtung und einer Telemetrieeinrichtung im Querschnitt;
- Figur 5: eine schematische Darstellung eines Wirbelkörpers mit einem hieran befestigten Implantat; und
- Figur 6: ein anderes Bauteil zum Montieren an einem Implantat mit einer Sensoreinrichtung und einer Telemetrieeinrichtung im Querschnitt.

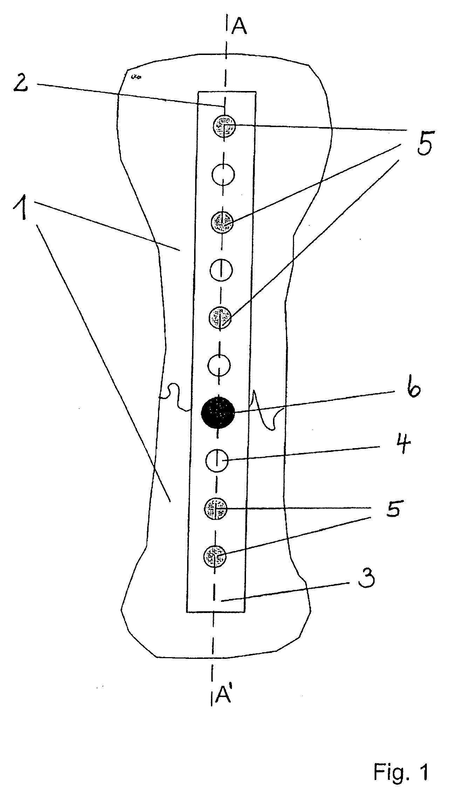

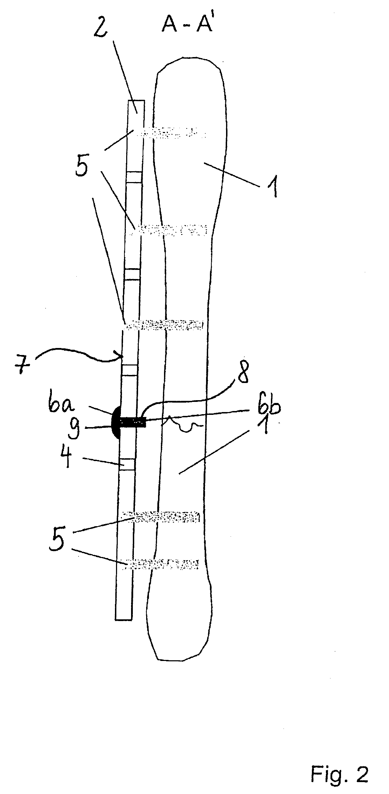

Figur 1 zeigt ein Knochenfragment 1 mit einem hieran befestigten Implantat 2, bei dem es sich um eine Schiene 3 mit mehreren Öffnungen 4 handelt, die als Durchgangsbohrungen ausgeführt sind. In einem Teil der Öffnungen 4 sind Schrauben 5 angeordnet, die zur Befestigung des Implantats 2 an dem Knochenfragment 1 dienen. Zu diesem Zweck sind die Schrauben 5 in das Knochenfragment 1 eingeschraubt, was sich deutlicher aus Figur 2 ergibt, welche einen Querschnitt durch das Knochenfragment 1 und das Implantat 2 nach Figur 1 entlang einer Linie AA' zeigt.

Ein Teil der zur Montage des Implantats 2 nutzbaren Öffnungen 4 in der Schiene 3 werden nicht zum Befestigen mittels der Schrauben 5 genutzt. In eine dieser nicht genutzten Öffnungen ist ein Bauteil 6 eingeführt. Das Bauteil 6 kann hierbei in die Schiene 3 eingesteckt oder eingeschraubt sein. Ein Kopfteil 6a des Bauteils 6 ist auf einer Außenfläche 7 der Schiene 3 angeordnet. Ein sich von dem Kopfteil 6a erstreckendes Basisteil 6b des Bauteils 6 erstreckt sich durch die Öffnung in der Schiene 3 hindurch, so daß ein Endabschnitt 8 des Basisteils 6b dem Knochenfragment 1 gegenüberliegend angeordnet ist. Ein gegenüberliegender Endabschnitt 9 im Bereich des Kopfteils 6a ist von dem Knochenfragment 1 entfernt und im wesentlichen frei auf der Außenfläche 7 der Schiene 3 plaziert.

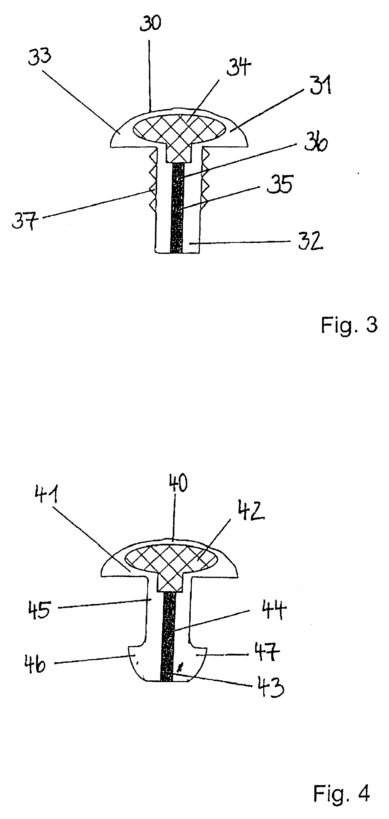

Ausführungsformen des Bauteils 6 sind in den Figuren 3 und 4 detaillierter dargestellt. Bei der Ausführungsform eines Bauteils 30 nach Figur 3 weist das Bauteil 30 im gezeigten Längsschnitt einen pilzförmigen Querschnitt mit einem Kopfteil 31 und einem Basisteil 32 auf. Das Bauteil 30 umfaßt ein Grundbauteil 33, in welchem eine Telemetrieeinrichtung 34 und eine Sensoreinrichtung 35 angeordnet sind. Die Sensoreinrichtung 35 und die Telemetrieeinrichtung 34 sind über eine Datenübertragungseinrichtung 36 miteinander verbunden. Mit Hilfe der Sensoreinrichtung 35 können Meßdaten für eine oder mehrere physikalische Meßgröße erfaßt werden, beispielsweise pH-Wert, Sauerstoffpartialdruck, Druck, Dehnung, Beschleunigung, Proteinkonzentration und/oder Spannung. Sensoren, die als solche bekannt sind und für die Integration in das Grundbauteil 33 geeignet sind, kann der Fachmann je nach auszuführender Meßanwendung auswählen. Mit Hilfe der Sensoreinrichtung 35 und der Telemetrieeinrichtung 34 ist eine telemetrische Meßsensoreinheit gebildet. Bei der Sensoreinrichtung 35 handelt es sich beispielsweise um einen Meßchip, einen Thermistor, einen ASIC oder dergleichen.

Die telemetrische Meßsensoreinheit kann als aktives Modul gebildet sein, welches über eine eigenen Energieversorgung verfügt, eine Batterie oder/und einen Akkumulator, die/der zumindest zeitweise die notwendige Energie zur Verfügung stellt. Sie kann auch als passives Modul ausgeführt sein, bei dem die benötigte Energie für die Sensoreinrichtung 35 und die Telemetrieeinrichtung 34 bedarfsweise von außen über eine kabellose Verbindung eingekoppelt wird.

Die in der Sensoreinrichtung 35 erzeugten Meßdaten für die Meßgröße(n) werden von der Sensoreinrichtung 35 über die Datenübertragungsverbindung 36 an die Telemetrieeinrichtung 34 übertragen. Die Telemetrieeinrichtung 34 verfügt über geeignete Sende-/Empfangsmittel, um die Meßdaten auf Anfrage kabellos an eine Auswerteeinrichtung (nicht dargestellt) zu übertragen. Die Datenübertragung kann mit Hilfe Auslesens der Daten ausgeführt werden, wozu ein externes Lesesystem (nicht dargestellt) genutzt wird. Das Lesesystem dient dazu, die Daten aus der Sensoreinrichtung 35 direkt oder über die Telemetrieeinrichtung 34 auf kabellosem, telemetrischem Weg auszulesen und gegebenenfalls einem sogenannten "Datalogger" oder einer Auswerteeinheit zuzuführen. Es kann vorgesehen sein, daß das Lesesystem in der Lage ist, eine Energieversorgung für die telemetrische Meßsensoreinheit bereitzustellen. Im Fall einer eigenen Energieversorgung der telemetrische Meßsensoreinheit, beispielsweise in Form einer Batterie oder eines Akkumulators, kann diese mit Hilfe des Lesesystems über eine kabellose Verbindung aufgeladen werden.

Die elektronischen Komponenten des Systems umfassen einen Signalkonditionierungschip (z. B. ASIC) mit AD-Wandler und Filterung, eine digitale Schnittstelle zur Rechnerankopplung, einem Transponder-IC mit integriertem Prozessor, ein Speichermedium, eine Echtzeituhr, eine Antennenspule und gegebenenfalls eine Energiequelle mit Powermanagement. Der Signalkonditionierungschip/ASIC ermöglicht eine elektronische Korrektur der individuellen Sensorfehler und gestattet somit eine hochpräzise Messung der Vitalparameter.

Das Gehäuse für die Telemetrie-Einheit besteht aus einem biokompatiblen polymeren Werkstoff, mit und ohne Beschichtung. Die Form und die technische Ausführung ist abhängig vom Einsatzort bzw. der Anwendung. Im Rahmen des Powermanagements kann ein Sleep-Modus aktiviert werden zur Reduzierung des Energieverbrauchs oder bei fehlender Energieversorgung.

Auf diese Weise ist es möglich, nach dem Anordnen des Bauteils 30 in einem Implantat, beispielsweise dem in Figur 1 gezeigten Implantat 2, und der Implantation des Implantats im Körper eines Lebewesens Meßdaten zu erfassen und zu einer Auswerteeinrichtung außerhalb des Körpers des Lebewesens zu übertragen. Die Übertragung derartiger Meßdaten ist als solche in Verbindung mit Sensoren im Körper eines Lebewesens bekannt und wird hier deshalb nicht weiter erläutert.

Gemäß Figur 3 ist an dem Basisteil 32 ein Gewindeabschnitt 37 vorgesehen, der dazu dient, daß Bauteil 30 bei der Montage an dem Implantat in die Implantatausnehmung einzuschrauben.

Figur 4 zeigt einen Querschnitt eines weiteren Bauteils 40, welches beispielsweise als eine Ausführungsform für das Bauteil 6 in der Anordnung nach Figur 1 verwendet werden kann. Im Grundbauteil 41 des Bauteils 40 sind eine Telemetrieeinrichtung 42 sowie eine Sensoreinrichtung 43 vorgesehen. Zur Übertragung von mit Hilfe der Sensoreinrichtung 43 erzeugter Meßdaten an die Telemetrieeinrichtung 42 ist wiederum eine Datenübertragungsverbindung 44 vorgesehen. Im Unterschied zu der Ausführungsform nach Figur 3 verfügt das Bauteil 40 im Bereich des Basisteils 45 nicht über einen Gewindeabschnitt. Es ist vielmehr ein verbreitertes Fußteil 46 vorgesehen. Wird das Bauteil 40 beim Anordnen in einer Implantatausnehmung mit dem Fußteil 46 zuerst in die Implantatausnehmung eingeführt, so drücken überstehende Seitenabschnitte 47 des Fußteil 46 gegen die Wandung der Implantatausnehmung, wodurch eine Klemmwirkung zum Befestigen des Bauteils 40 an dem Implantat entfaltet wird. Hierbei kann vorgesehen sein, daß das Grundbauteil 41 aus einem in gewissem Umfang nachgebenden Material gebildet ist, so daß die überstehenden Seitenabschnitte 47 beim Einführen in die Implantatausnehmung zum Teil eingedrückt werden.

In Abhängigkeit von den Anwendungsfällen kann der Fachmann zwischen unterschiedlichen Materialien für die Grundbauteile 41 bzw. 33 auswählen. Während bei der Ausführungsform des Bauteils 30 ein eher festes Material zu nutzen ist, beispielsweise ein Metall, um den Gewindeabschnitt 37 auszubilden, kann für die Ausführungsform des Bauteils 40 ein Material gewählt werden, das in gewissem Umfang elastisch oder nicht elastisch verformbar ist, beispielsweise ein Kunststoff. Wenn das Bauteil 40 zum Teil durch eine als Durchbruch ausgeführte Implantatausnehmung hindurch gesteckt wird, verhindert das Fußteil ein Herausrutschen des Bauteils 40.

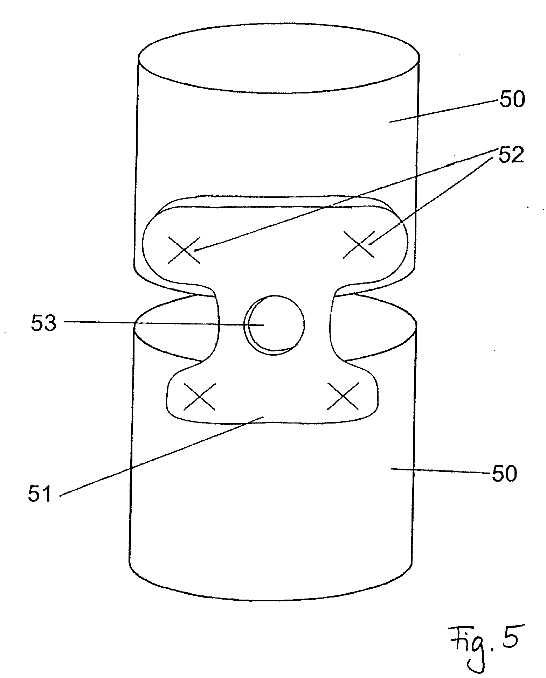

Figur 5 zeigt einen Wirbelkörper 50, an dem ein Implantat 51 mittels Schrauben 52 befestigt ist. Eine Öffnung 53 in dem Implantat 51 ist ungenutzt, so daß in dieser Öffnung 53 ein Bauteil angeordnet werden kann, wie es beispielhaft unter Bezugnahme auf die Figuren 3 und 4 beschrieben wurde.

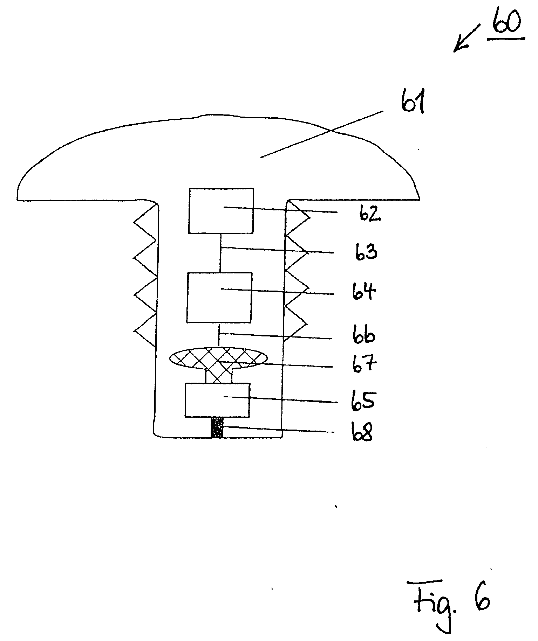

Figur 6 zeigt ein anderes Bauteil 60 im Querschnitt, welches insbesondere als eine Ausführungsform für das Bauteil 6 in der Anordnung nach Figur 1 verwendet werden kann. In eine Kapselung 61 ist eine Applikationseinheit 62 integriert. Die Applikationseinheit 62 empfängt mit Hilfe einer Sensoreinheit (nicht dargestellt) erfaßte Meßsignale über eine drahtlose oder drahtgebundene Verbindung zur elektronischen Datenübertragung. Über eine Signalleitung 63 ist die Applikationseinheit 62 mit einer Steuereinheit 64 verbunden. In der Steuereinheit 64 sind in Form elektronischer Daten typische Kennlinien für die mit Hilfe der Sensoreinheit zu erfassenden Meßsignale in einem Speicher gespeichert. Die typischen Kennlinien können von außen über eine Telemetrie-Anbindung der Sensoreinheit durch geänderte Kennlinien ersetzt werden. In der Steuereinheit 64 werden zum Ansteuern einer Pumpeinrichtung 65, die über eine Steuerleitung 66 mit der Steuereinheit 64 verbunden ist, Steuersignale auf Basis eines Istwert/Sollwert-Vergleiches der erfaßten Meßsignale mit dem für die typischen Kennlinien gespeicherten Daten erzeugt. In Abhängigkeit von den Steuersignalen wird dann mit Hilfe der Pumpeinrichtung 65 ein Wirkstoff aus einem Wirkstoffvorrat 67 über eine verschließbare Öffnung 68 abgegeben. Der Wirkstoff ist eine chemische Verbindung beliebiger Art, die zur Erzielung einer medizinischen Wirkung an die Umgebung des anderen Bauteils 60 abgegeben wird, beispielsweise um einen Heilungsprozeß zu beeinflußen. Aufgrund der Wirkstoffabgabe kann eine Änderung der erfaßten Meßsignale an der Sensoreinheit erwartet werden. Die geänderten Meßsignale an der Sensoreinheit werden nach Übertragung an die Applikationseinheit 62 dann wiederum in der Steuereinheit 64 mit Sollwerten verglichen, so daß eine Art Regelkreis für die Wirkstoffabgabe gebildet ist. Der Fachmann kann zur Implementierung des anderen Bauteils 60 nach Figur 6 geeignete Mikroprozessortechnik sowie Mikrosystemtechnik, beispeilsweise Mikropumptechnik auswählen, die in verschiedenen Formen bekannt ist.

Die Wirkstofffreigabe kann auch passiv erfolgen, zum Beispiel mittels eines bioabbaubarem (Matrix)systems oder der Freigabe durch Diffusion aus einer Matrix.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

### Bevorzugte Ausführungsformen sind in den folgenden Absätzen definiert

1. Bauteil zum Anordnen an einem Implantat, mit:
   - einem Grundbauteil;
   - mindestens einer in dem Grundbauteil angeordneten Sensoreinrichtung zum Erfassen einer Meßgröße und zum Erzeugen von Meßdaten für die erfaßte Meßgröße;
   - einer in dem Grundbauteil angeordneten Telemetrieeinrichtung zum Senden und/oder Empfangen von Daten; und
   - einer Datenübertragungsverbindung zwischen der mindestens einen Sensoreinrichtung und der Telemetrieeinrichtung zum Übertragen von Daten zwischen der mindestens einen Sensoreinrichtung und der Telemetrieeinrichtung, wobei die Daten die Meßdaten umfassen;
   wobei an dem Grundbauteil Montagemittel zum lösbaren Montieren des Grundbauteils in einer Implantatausnehmung eines Implantats gebildet sind.
2. Bauteil nach Absatz 1, dadurch **gekennzeichnet,** daß die Montagemittel einen Montageabschnitt zum wenigstens teilweisen Einführen in die Implantatausnehmung umfassen.
3. Bauteil nach Absatz 2, dadurch **gekennzeichnet,** daß im Bereich des Montageabschnitts eine Gewindeabschnitt zum Einschrauben des Grundbauteils in die Implantatausnehmung gebildet ist.
4. Bauteil nach einem der vorangehenden Absätze , dadurch **gekennzeichnet,** daß das Grundbauteil im Längsschnitt einen im wesentlichen T-förmigen Querschnitt mit einem Kopfteil und einem Basisteil aufweist.
5. Bauteil nach einem der vorangehenden Absätze , dadurch **gekennzeichnet,** daß die mindestens eine Sensoreinrichtung im Bereich eines Endabschnitts des Grundbauteils und die Telemetrieeinrichtung im Bereich eines gegenüberliegenden Endabschnitts des Grundbauteils angeordnet sind.
6. Bauteil nach Absatz 4 oder 5, dadurch **gekennzeichnet,** daß die Telemetrieeinrichtung im wesentlichen in dem Kopfteil des Grundbauteils angeordnet ist.
7. Bauteil nach einem der vorangehenden Absätze , dadurch **gekennzeichnet,** daß an dem Grundbauteil ein Aufnahmeraum zum Aufnehmen eines Wirkstoffs gebildet ist, wobei der Aufnahmeraum mit einer Öffnung zum Abgeben des Wirkstoffs nach außen in Verbindung steht.
8. Bauteil nach Absatz 7, **gekennzeichnet** durch eine Abgabeeinrichtung zum kontrollierten Abgeben des Wirkstoffs aus dem Aufnahmeraum durch die Öffnung.
9. Bauteil nach Absatz 8, dadurch **gekennzeichnet,** daß die Abgabeeinrichtung eine Pumpeinrichtung zum Pumpen einer Menge des Wirkstoffs aus dem Aufnahmeraum durch die Öffnung umfaßt.
10. Bauteil nach Absatz 8 oder 9, dadurch **gekennzeichnet,** daß die Abgabeeinrichtung einen Öffnungsmechanismus zum Öffnen/Schließen der Öffnung umfaßt.
11. Bauteil nach einem der Absätze 8 bis 10, dadurch **gekennzeichnet,** daß die Abgabeeinrichtung über einer weitere Datenübertragungsverbindung zum Übertragen von Daten mit der Telemetrieeinrichtung verbunden ist.
12. Bauteil nach einem der Absätze 8 bis 11, **gekennzeichnet** durch eine Steuereinheit, die mit der mindestens einen Sensoreinrichtung und der Abgabeeinrichtung verbunden ist, um die Erfassung der Meßdaten mit Hilfe der mindestens einen Sensoreinrichtung und die Abgabe des Wirkstoffs mit Hilfe der Abgabeeinrichtung gemeinsam zu steuern.
13. Stützimplantat, insbesondere Platte oder Schiene aus einem Material mit hoher Steifigkeit, dadurch **gekennzeichnet,** daß an dem Implantat ein Bauteil nach einem der Absätze 1 bis 12 angeordnet ist.
14. Stützimplantat nach Absatz 13, dadurch **gekennzeichnet,** daß das Bauteil in einer Implantatausnehmung angeordnet ist.
15. Stützimplantat nach Absatz 14, dadurch **gekennzeichnet,** daß die Implantatausnehmung eine beim Implantieren nutzbare Montageausnehmung zum Aufnehmen von Implantat-Befestigungsmitteln ist.
16. Stützimplantat nach Absatz 15, dadurch **gekennzeichnet,** daß die Implantatausnehmung einen Innengewindeabschnitt aufweist.
17. Ersatzimplantat, insbesondere künstliches Hüft-, Knie- oder Schultergelenk, dadurch **gekennzeichnet,** daß an dem Implantat ein Bauteil nach einem der Absätze 1 bis 12 angeordnet ist.
18. Ersatzimplantat nach Absatz 17, dadurch **gekennzeichnet,** daß das Bauteil in einer Implantatausnehmung angeordnet ist.
19. Ersatzimplantat nach Absatz 18, dadurch **gekennzeichnet,** daß die Implantatausnehmung eine beim Implantieren nutzbare Montageausnehmung zum Aufnehmen von Implantat-Befestigungsmitteln ist.
20. Ersatzimplantat nach Absatz 19, dadurch **gekennzeichnet,** daß die Implantatausnehmung einen Innengewindeabschnitt aufweist.

## Patentansprüche

1. Bauteil zum Anordnen an einem Implantat (2; 51), mit:
- einem Grundbauteil (33; 41);
- mindestens einer in dem Grundbauteil (33; 41) angeordneten Sensoreinrichtung (35; 43) zum Erfassen einer Messgröße und zum Erzeugen von Messdaten für die erfasste Messgröße;
- einer in dem Grundbauteil (33; 41) angeordneten Telemetrieeinrichtung (34; 42) zum Senden und/oder Empfangen von Daten;
- einer Datenübertragungsverbindung (36; 44) zwischen der mindestens einen Sensoreinrichtung (35; 43) und der Telemetrieeinrichtung (34; 42) zum Übertragen von Daten zwischen der mindestens einen Sensoreinrichtung (35; 43) und der Telemetrieeinrichtung (34; 42), wobei die Daten die Messdaten umfassen; und
- einem in dem Grundbauteil (33; 41) gebildeten Aufnahmeraum (67) zum Aufnehmen eines Wirkstoffs, wobei der Aufnahmeraum (67) mit einer Öffnung (68) zum Abgeben des Wirkstoffs nach außen in Verbindung steht,
wobei an dem Grundbauteil (33; 41) Montagemittel (37; 47) zum lösbaren Montieren des Grundbauteils (33; 41) in einer über einem Zielbereich eines Knochens positionierbaren Implantatausnehmung (4; 53) eines Implantats (2; 51) gebildet sind, wobei das Grundbauteil (33; 41) eingerichtet ist, derart in der Implantatausnehmung (4; 53) montiert zu werden, dass die Öffnung (68) zum Abgeben des Wirkstoffs über dem Zielbereich des Knochens angeordnet ist, um den Wirkstoff an den Zielbereich des Knochens abzugeben.

2. Bauteil nach Anspruch 1, wobei die Montagemittel einen Montageabschnitt (37; 47) zum wenigstens teilweisen Einführen in die Implantatausnehmung (4; 53) umfassen.

3. Bauteil nach Anspruch 2, wobei im Bereich des Montageabschnitts ein Gewindeabschnitt (37) zum Einschrauben des Grundbauteils (33) in die Implantatausnehmung (4; 53) gebildet ist.

4. Bauteil nach einem der Ansprüche 1 bis 3, wobei das Grundbauteil (33; 41) im Längsschnitt einen im Wesentlichen T-formigen Querschnitt mit einem Kopfteil (6a) und einem Basisteil (6b) aufweist.

5. Bauteil nach einem der Ansprüche 1 bis 4, wobei die mindestens eine Sensoreinrichtung (35; 43) im Bereich eines Endabschnitts des Grundbauteils (33; 41) und die Telemetrieeinrichtung (34; 42) im Bereich eines gegenüberliegenden Endabschnitts des Grundbauteils (33; 41) angeordnet sind.

6. Bauteil nach Anspruch 4 oder 5, wobei die Telemetrieeinrichtung (34; 42) im Wesentlichen in dem Kopfteil (6a) des Grundbauteils (33; 41) angeordnet ist.

7. Bauteil nach einem der Ansprüche 1 bis 6, des Weiteren umfassend eine Abgabeeinrichtung zum kontrollierten Abgeben des Wirkstoffs aus dem Aufnahmeraum (67) durch die Öffnung (68).

8. Bauteil nach Anspruch 7, wobei die Abgabeeinrichtung eine Pumpeinrichtung (65) zum Pumpen einer Menge des Wirkstoffs aus dem Aufnahmeraum (67) durch die Öffnung (68) umfasst.

9. Bauteil nach Anspruch 7 oder 8, wobei die Abgabeeinrichtung einen Öffnungsmechanismus zum Öffnen/Schließen der Öffnung (68) umfasst.

10. Bauteil nach einem der Ansprüche 7 bis 9, wobei die Abgabeeinrichtung über eine weitere Datenübertragungsverbindung (63) zum Übertragen von Daten mit der Telemetrieeinrichtung (34; 42) verbunden ist.

11. Bauteil nach einem der Ansprüche 7 bis 10, des Weiteren umfassend eine Steuereinheit (64), die mit der mindestens einen Sensoreinrichtung und der Abgabeeinrichtung verbunden ist, um die Erfassung der Messdaten mit Hilfe der mindestens einen Sensoreinrichtung und die Abgabe des Wirkstoffs mit Hilfe der Abgabeeinrichtung gemeinsam zu steuern.

12. Stützimplantat, insbesondere Platte oder Schiene aus einem Material mit hoher Steifigkeit, mit mehreren, beim Implantieren nutzbaren Implantatausnehmungen (4; 53) zum Aufnehmen von Implantat-Befestigungsmitteln (5; 52), wobei ein Bauteil (6; 30; 40; 60) nach einem der Ansprüche 1 bis 11 in einer der Implantatausnehmungen (4; 53) angeordnet ist.

13. Stützimplantat nach Anspruch 12, **dadurch gekennzeichnet, dass** die Implantatausnehmung (4; 53) einen Innengewindeabschnitt aufweist.

14. Ersatzimplantat, insbesondere künstliches Hüft-, Knie- oder Schultergelenk, mit mehreren, beim Implantieren nutzbaren Implantatausnehmungen zum Aufnehmen von Implantat-Befestigungsmitteln, wobei ein Bauteil (6; 30; 40; 60) nach einem der Ansprüche 1 bis 11 in einer der Implantatausnehmungen angeordnet ist.

15. Ersatzimplantat nach Anspruch 14, wobei die Implantatausnehmung einen Innengewindeabschnitt aufweist.
